# EUROPEAN PATENT APPLICATION

(11) **EP 4 696 684 A1**
(43) Date of publication of application: **18.02.2026**
(21) Application number: 24194769.6
(22) Date of filing: 15.08.2024
(51) Int. Cl.: C07D 307/44, C07D 307/50

(54) **DEHYDRATION AND HYDROGENATION PROCESS OF BIOMASS FEEDSTOCK FOR PREPARING FURANIC INTERMEDIATES**

(71) Applicant: Nederlandse Organisatie voor toegepast- natuurwetenschappelijk onderzoek TNO, 2595 DA 's-Gravenhage (NL)
(72) Inventor: CROCKATT, Marc, 2595 DA 's-Gravenhage (NL); VAN DER WAAL, Jan Cornelis, 2595 DA 's-Gravenhage (NL); KÖNST, Paul Mathijs, 2595 DA 's-Gravenhage (NL)
(74) Representative: V.O.

(57) **Abstract**

The invention is directed to a process for preparing a furanic intermediate, said process comprising a dehydration step of C5- and/or C6-carbohydrates comprised in a biomass feedstock solution, in the presence of an acid catalyst to form a furanic intermediate, wherein said dehydration step is carried out in a fluid comprising an aqueous solvent and an organic solvent, wherein the organic solvent has a log(water solubility in ppm) at room temperature in the range of -4.0 to -1.5 and a boiling point of less than 210 °C.

## Description

The invention relates to a process for preparing dehydration and hydrogenation products from biomass feedstock containing C5- and/or C6-carbohydrates.

Most commercial processes for converting C5- and/or C6-carbohydrates by dehydration and hydrogenation make use of a two-step process, wherein dehydration to a furanic intermediate is conducted in water, while hydrogenation of the furanic intermediate, herein also referred to as the dehydration product, is conducted in an organic solvent. In such a two-step process, the dehydration product formed in the first step is typically isolated or extracted into a different, organic solvent and purified before the second step of hydrogenation is conducted.

Processes are currently being researched wherein dehydration is conducted in a biphasic reaction system containing an aqueous and an organic phase. Dehydration of sugar takes place in the aqueous phase and the dehydration product formed, a furanic intermediate, is continuously extracted into the organic phase. The advantage of this continuous extraction of dehydration product into an organic phase is that the furanic intermediate is more stable in the organic phase compared to the aqueous phase under the reaction conditions of the dehydration, which include high temperatures and highly acidic environment due to the presence of a watersoluble acid catalyst. Under these conditions, the furanic intermediate may *inter alia* degrade via ring-opening and/or polymerization reactions and/or reactions with the starting sugar. After the dehydration reaction, the furanic intermediate in the organic phase can be subjected to hydrogenation in the same or in a second reactor.

For instance, in Ordomsky *et al.* a process is described wherein both dehydration and hydrogenation take place in one and the same biphasic reactor system. In this process, dehydration of xylose takes place in the aqueous phase and hydrogenation of furfural takes place in the organic phase and/or at the interface between the aqueous and the organic phase. As the organic phase, either 2-methyltetrahydrofuran, 1-butanol or cyclohexane was used.

US2008033188A1 describes a process for the production of furan derivatives in a biphasic system, after which the furan derivative is evaporated and subjected to hydrogenolysis in the vapor phase. In this process, a two-phase reactor system is used, wherein the sugar is dehydrated in an aqueous phase and the furan derivative is continuously extracted into an organic phase.

US8389749B2 describes a process to make furan derivative compounds such as furfuryl alcohol from xylose by using the organic solvent alkylphenol in a biphasic system. First, the xylose is dehydrated in the biphasic system and next a hydrogenation reaction with the same organic alkylphenol solvent is carried out.

The use of the same organic solvent as present in the biphasic system is desirable, because this prevents the need for a process step in between both reactions that involves evaporation of either the solvent or the furfural derivative, resulting in a more time, energy and cost-effective process. This is especially favorable when using biomass feedstock with low carbohydrate concentrations, which could require evaporating large amounts of solvent.

However, the solvents used in the processes described above generally lead to poor yields and selectivities. Therefore, there remains a need for optimizing the dehydration and hydrogenation process to a more efficient process, while achieving a good yield and selectivity.

Surprisingly, the present inventors found a correlation between the water-solubility of the organic solvent used in both the dehydration and hydrogenation reactions and the yields and selectivities for both reactions. Accordingly, in a first aspect, the invention is directed to a process for preparing a furanic intermediate, said process comprising a dehydration step of C5- and/or C6-carbohydrates comprised in a biomass feedstock solution, in the presence of an acid catalyst to form one or more furanic intermediates, wherein said dehydration step is carried out in a fluid comprising an aqueous solvent and an organic solvent, wherein the organic solvent has a log(water solubility in ppm) in the range of -4.0 to -1.5 at 20 °C and a boiling point of less than 210 °C.

The invention is particularly suitable for processes wherein the biomass feedstock solution is a dilute C5- and/or C6-carbohydrate solution from a biorefinery, wherein the concentration of carbohydrate is less than 10%, such as for example in the range of 1-7%, based on the total weight of biomass feedstock solution.

The biomass feedstock comprises hemicellulose and/or cellulose, preferably at least hemicellulose, which comprise C5- and C6-carbohydrates such as xylose, arabinose, mannose, galactose and glucose. The biomass feedstock preferably comprises C5-carbohydrates, more preferably xylose. C5-carbohydrates are preferred, because they are derived from the non-edible fraction of biomass.

The inventors found that the water-solubility of the organic solvent should not be too high. Without wishing to be bound by theory, the present inventors believe that water present in the organic phase of the biphasic system may degrade furfural and/or that the organic solvent present in the water phase increases the solubility of the furfural in the aqueous phase, thereby exposing it to water and acid and decreasing its stability. Thus, the organic solvent preferably has a low water solubility. Further, the organic solvent preferably is sufficiently hydrophobic to efficiently extract the furfural derivatives. On the other hand, the organic solvent preferably is sufficiently hydrophilic to achieve good selectivity in the hydrogenation step. The hydrophobic or hydrophilic character of a solvent is also reflected by its water solubility. Based on these contradictory requirements, the ideal range of water solubility at room temperature for the organic solvent was determined. To express these factors, the logarithm of the water solubility in ppm of the organic solvent in water (herein abbreviated as log(water solubility in ppm), or simply, log(water solubility) can be used. The water solubility is herein expressed in ppm, which corresponds to the solubility of the organic solvent in mg per L, wherein 1 ppm equals 1 mg/L, at 20 °C and 1 bar. The log(water solubility in ppm) of suitable organic solvents was found to fall within the range of about -4.0 to about -1.5, preferably between -3.5 and -1.65. A log(water solubility) of -4.0 corresponds to a relatively hydrophobic organic solvent, whereas a log(water solubility) of -1.5 corresponds to a relatively hydrophilic organic solvent.

The inventors found that the boiling point of a solvent suitable for both the dehydration and hydrogenation reaction is preferably also taken into account. A high boiling point complicates recycling of the organic solvent. For example, the organic solvent after the hydrogenation step may be recycled for use in the dehydration reaction. Therefore, an ideal solvent should preferably have a boiling point of below 210 °C. On the other hand, if the solvent has a low boiling point, the reaction product cannot easily be separated by distillation. In addition, solvents with a low boiling point can lead to undesirably high pressures in the reactor. Therefore, preferably, the organic solvent has a boiling point between 70 and 210 °C, even more preferably between 100 and 180 °C. All boiling points are given herein at standard, 1 bar, pressure.

The dehydration reaction to form the furanic intermediate is typically carried out at a temperature between 140 and 220 °C, preferably between 160 and 200 °C. The dehydration reaction may be heated between 2 and 60 minutes, preferably between 15 and 45 minutes. Typically, the dehydration reaction is performed at a pH of less than 2.0 and above 0.0, preferably between 0.5 and 1.5, and with an aqueous to organic solvent ratio of between 5:1 and 1:5, preferably between 3:1 and 1:3. Optionally, additives to improve phase separation, including salts such as sodium chloride or sodium sulfate, or to improve stability of the sugar substrates or furanic products, such as boronic acid derivatives, may be added.

The organic solvent is preferably selected from the group consisting of thioethers, ethers, esters, alcohols, carbonates, carboxylic acids, hydrocarbons, and combinations thereof, preferably from the group consisting of ethers, esters, alcohols, and combinations thereof. Advantageously, ethers, esters and alcohols are more stable under the dehydration and hydrogenation conditions compared to acids, alcohols, esters and ketones, and are therefore preferred.

From a sustainability perspective it is desirable to use an organic solvent that can be produced from biomass via bio-based conversion. Examples of such organic solvents include alkyl esters, alkyl ethers and alcohols. In particular aliphatic esters and ethers having 1 to 5 carbon atoms at either side of the ester or ether bond. Symmetrical ethers having 1 to 5 carbon atoms at either side of the ether bond are especially preferred. This number of carbon atoms generally corresponds to the non-edible fraction of biomass, *e.g.* C5-carbohydrates, that can be converted to these solvents.

The organic solvent is preferably a solvent selected from the list provided in Table 1.

**Table 1**

| **Common name** | **IUPAC name** | **b.p.* (°C)** | **Log(water solubility in ppm)** |
|---|---|---|---|
| Ethyl propionate | Ethyl propanoate | 99.1 | -1.72 |
| Propyl acetate | Propyl acetate | 101.5 | -1.72 |
| Diethylcarbonate | Diethyl carbonate | 126 | -1.73 |
| Ethyl propyl ether | 1-Ethoxypropane | 63.2 | -1.74 |
| Methyl butyrate | Methyl butanoate | 102.8 | -1.82 |
| Methylfuroate | Methyl 2-furoate | 185 | -1.88 |
| Cyclopentyl methyl ether | Methoxycyclopentane | 106 | -1.96 |
| Hexanoic acid | Hexanoic acid | 205.2 | -1.99 |
| Ethylene glycol butyl ether acetate | 2-Butoxyethyl acetate | 192 | -2.05 |
| Diisopropyl ether | 2-[(Propan-2-yl)oxylpropane | 68.5 | -2.06 |
| Butyl acetate | Butyl acetate | 126.1 | -2.08 |
| Dimethyltetrahydrofuran | 2,5-Dimethyltetrahydrofuran | 92 | -2.08 |
| tert-Butyl acetate | tert-Butyl acetate | 97 | -2.08 |
| Isobutylacetate | 2-Methylpropyl acetate | 116.5 | -2.20 |
| sec-Butylacetate | Butan-2-yl acetate | 112 | -2.21 |
| 1-Hexanol | Hexan-1-ol | 157.6 | -2.23 |
| Dipropylether | 1-Propoxypropane | 90 | -2.31 |
| Ethyl butyrate | Ethyl butanoate | 121 | -2.31 |
| Propyl propionate | Propyl propanoate | 123 | -2.37 |
| Diethyl thioether | (Ethylsulfanyl)ethane | 92.1 | -2.50 |
| Dimethyl thioether | Dimethylsulfide | 109.8 | -2.52 |
| tert-Amyl methyl ether | 2-Methoxy-2-methylbutane | 86.3 | -2.58 |
| Methylbenzoate | Methyl benzoate | 199 | -2.68 |
| Isoamyl acetate | 3-Methylbutyl acetate | 142.5 | -2.70 |
| Ethylene glycol dibutyl ether | 1-(2-butoxyethoxy)butane | 203.3 | -2.70 |
| Pentyl acetate | Pentyl acetate | 149.2 | -2.77 |
| 1-Heptanol | Heptan-1-ol | 176.4 | -2.78 |
| 2-Octanol | Octan-2-ol | 175 | -2.89 |
| Isobutyl isobutyrate | 2-Methylpropyl 2-methylpropanoate | 148.6 | -3.00 |
| 2-Ethylhexanol | 2-Ethylhexan-1-ol | 184.6 | -3.06 |
| 6-Methyl-1-heptanol | 6-Methylheptan-1-ol | 188 | -3.19 |
| 1-Octanol | Octan-1-ol | 195.1 | -3.27 |
| Hexyl acetate | Hexyl acetate | 171.5 | -3.29 |
| Butylbutyrate | Butyl butanoate | 166 | -3.30 |
| Dibutyl ether | 1-Butoxybutane | 140.2 | -3.52 |
| Ethylbenzene | Ethylbenzene | 136.1 | -3.77 |
| 1,2-Xylene | 1,2-Xylene | 138.3 | -3.79 |
| m-Xylene | 1,3-Xylene | 139.1 | -3.79 |
| Cyclopentane | Cyclopentane | 49.3 | -3.81 |
| 1-Pentene | Pent-1-ene | 29.9 | -3.83 |

| | | | |
|---|---|---|---|
| *b.p. stands for boiling point at 20 °C and 1 bar. | | | |

In particularly preferred embodiments, the organic solvent is selected from the list consisted of dibutyl ether, butyl acetate, butyl butyrate, pentyl acetate, propyl propionate, ethyl butyrate, methyl butyrate, propyl acetate, diethyl carbonate, and combinations thereof, as these solvents gave good results. The organic solvent is most preferably dibutyl ether, as this gave particularly good results.

In embodiments wherein the biomass feedstock solution comprises C5-carbohydrates, these C5-carbohydrates are converted in the dehydration reaction into furfural. Similarly, in embodiments wherein the biomass feedstock solution comprises C6-carbohydrates, these C6-carbohydrates are converted into substituted furfurals, such as 5-hydroxymethylfurfural (abbreviated as 5-HMF), 5-methyl furfural, 5-chloromethylfurfural and/or alkyl ethers of 5-hydroxymethylfurfural. The substituted furfural formed can be influenced by the reaction conditions. For instance, alkyl ethers of 5-hydroxymethylfurfural can be formed if the dehydration reaction is carried out in the presence of a corresponding alcohol. Accordingly, the furanic intermediate formed in the present invention, comprises a compound selected from the group consisting of furfural, 5-methyl furfural, 5-hydroxymethyl furfural, 5-alkoxymethyl furfural, 5-chloromethylfurfural, and combinations thereof, wherein the alkoxy group of alkoxymethyl fufural preferably has 1 to 8 carbon atoms. The furanic intermediate formed preferably comprises furfural, because the biomass feedstock solution preferably comprises C5-carbohydrates.

In a further aspect, the present invention is directed to a two-step process comprising the dehydration step of C5- and/or C6-carbohydrates comprised in a biomass feedstock solution to form the furanic intermediate as described herein, followed by a hydrogenation step of the furanic intermediate to form a hydrogenation product. In this two-step process, the hydrogenation step is carried out in a fluid comprising at least the organic solvent that was used for the dehydration step.

It may be appreciated that the fluid in which the hydrogenation step is carried out can be the same fluid in which the dehydration step was carried out. Hence no intermediate isolation or separation is required following the dehydration step. This is preferred as it reduces process steps.

In another embodiment, the process for preparing a hydrogenation product can comprise a separation step following the dehydration step, wherein the organic solvent comprising at least part of the furanic intermediate is separated from the aqueous solvent, and wherein the separated organic solvent comprising at least part of the furanic intermediate is subsequently used in the hydrogenation step. In such an embodiment, the process may also comprise drying, filtering and/or washing, preferably with activated carbon, of the separated organic solvent in order to remove impurities before the hydrogenation step.

The hydrogenation reaction is typically carried out in the presence of both hydrogen gas, at pressures between for instance 1 and 100 bar, preferably between 30 and 80 bar, and a catalyst. The catalyst may be homogenous but is preferably heterogeneous, and preferably a noble metal, such as gold, platinum, ruthenium, rhodium, palladium, osmium, iridium, silver, copper, mercury, technetium, rhenium, arsenic, antimony, or bismuth, supported on an inert material, such carbon, alumina, silica, titania, zirconia, magnesia, alumino silicates/zeolites, calcium carbonate, sodium phosphate, barium sulfate or phosphate, cerium oxide, europium oxide, gadolinium oxide, montmorillonite, hydrotalcite, kaolinite or polymeric resins. The catalyst may optionally also contain non-noble metals in combination with the noble metal. The reaction is performed in the solvent that was used during the dehydration step, optionally diluted with more of the same of another solvent.

The hydrogenation reaction of the furanic intermediate can be carried out at temperatures between 100 and 260 °C, preferably between 160 and 200 °C. The hydrogenation reaction may be heated for between 0.5 and 300 minutes, preferably between 1 and 180 minutes. In a batch process with relatively low catalyst loading, several hours are generally required, but in a continuous process with a packed bed reactor, with relatively high catalyst loading, this can be reduced to a few minutes.

The step of hydrogenating the furanic intermediate generally leads to one or more products according to formula (I), 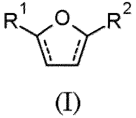 wherein
R¹ represents H, Me, CH₂OH, CH₂OR⁴, wherein R⁴ is a linear or branched alkyl group having 2-10 carbon atoms, or CH₂Cl; and
R² represents H, Me, CHO, CH₂OH, -C(O)CH₃, -CH₂O(O)CR³, -CH₂(furan-2-yl), -CH₂(oxolan-2-yl), -CH₂(5-methylfuran-2-yl), -CH₂(5-methyloxolan-2-yl), -CH₂(5-hydroxymethylfuran-2-yl), -CH₂(5-hydroxymethyloxolan-2-yl), - CH₂(5-[R⁵OCH₂]furan-2-yl), -CH₂(5-[R⁵OCH₂]oxolan-2-yl), -CH₂(5-chloromethylfuran-2-yl), or -CH₂(5-chloromethyloxolan-2-yl), wherein R³ and R₅ individually represent a linear or branched alkyl group having 2-10 carbon atoms.

It may be appreciated that the product that is formed depends on the hydrogenation reaction as well as on the starting material of the dehydration reaction, *i.e.* on the C5- and/or C6-carbohydrates comprised in a biomass feedstock solution. When the furanic intermediate is derived from C5-carbohydrates, the hydrogenation product may comprise a compound according to formula (I), 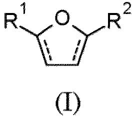 wherein
R¹ = H or Me; and
R² = H, Me, -CHO, -CH₂OH, -CH₂C(O)CH₃, -CH₂CH₂O(O)CR³, wherein R³ is a linear or branched C2- to C10-alkyl, -CH₂(furan-2-yl), -CH₂(oxolan-2-yl), - CH₂(5-methylfuran-2-yl), -CH₂(5-methyloxolan-2-yl), a C5-diol, or a C4- or C5-alcohol.

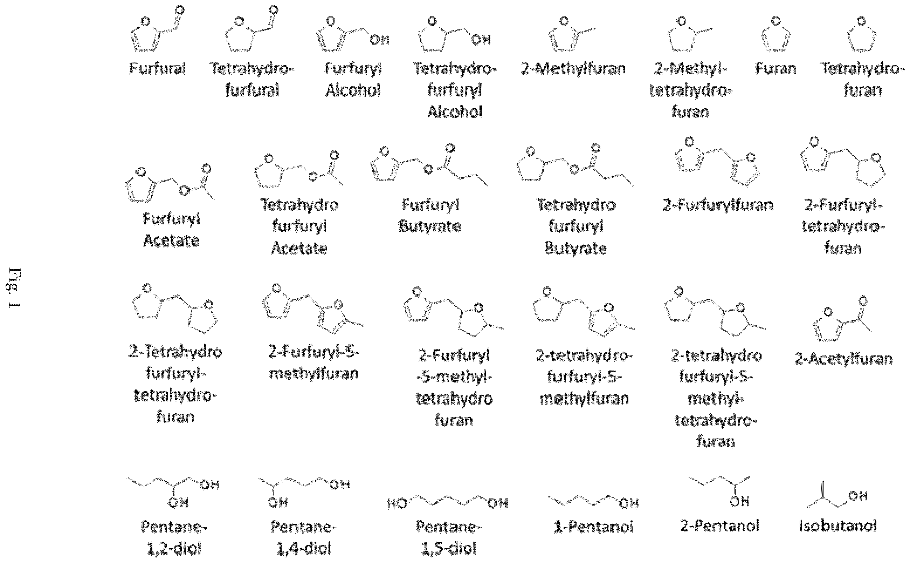

The product is preferably selected from the group consisting of furfural, tetrahydrofurfural, furfuryl alcohol, tetrahydrofurfuryl alcohol, 2-methylfuran, 2-tetrahydrofuran, furan, tetrahydrofuran, furfuryl acetate, tetrahydrofurfuruyl acetate, furfuryl butyrate, tetrahydrofurfuryl butyrate, 2-furfurylfuran, 2-furfuryltetrahydrofuran, 2-tetrahydrofurfuryltetrahydrofuran, 2-furfuryl-5-methylfuran, 2-furfuryl-5-methyltetrahydrofuran, 2-tetrahydrofurfuryl-5-methylfuran, 2-tetrahydrofurfuryl-5-methyltetrahydrofuran, 2-acetylfuran, pentane-1,2-diol, pentane-1,4-diol, pentane-1,5-diol, 1-pentanol, 2-pentanol, and isobutanol. The corresponding IUPAC name and chemical structure of each of these products is provided in Table 2 and Figure 1, respectively.

The product more preferably comprises 2-methylfuran and/or furfuryl alcohol, even more preferably 2-methylfuran.

**Table 2**

| **Name** | **IUPAC Name** |
|---|---|
| Furfural | 2-Furaldehyde |
| Tetrahydrofurfural | Oxolane-2-carbaldehyde |
| Furfuryl alcohol | 2-(Hydroxymethyl)furan |
| Tetrahydrofurfuryl alcohol | (Oxolan-2-yl)methanol |
| 2-Methylfuran | 2-Methylfuran |
| 2-methyltetrahydrofuran | 2 -Methyloxolane |
| Furan | Furan |
| Tetrahydrofuran | Tetrahydrofuran |
| Furfuryl acetate | (Furan-2-yl)methyl acetate |
| Tetrahydrofurfuryl acetate | (Oxolan-2-yl)methyl acetate |
| Furfuryl butyrate | (Furan-2-yl)methyl butanoate |
| Tetrahydrofurfuryl butyrate | (Oxolan-2-yl)methyl butanoate |
| 2-Furfurylfuran | 2,2'-methylenedifuran |
| 2-Furfuryltetrahydrofuran | 2-[(oxolan-2-yl)methyl]furan |
| 2-Tetrahydrofurfuryltetrahydrofuran | 2,2'-methylenebis(oxolane) |
| 2-Furfuryl-5-methylfuran | 2-[(furan-2-yl)methyl]-5-methylfuran |
| 2-Furfuryl-5-methyltetrahydrofuran | 2-[(5-methyloxolan-2- yl)methyl]furan |
| 2-Tetrahydrofurfuryl-5-methylfuran | 2-methyl-5-[(oxolan-2-yl)methyl]furan |
| 2-Tetrahydrofurfuryl-5-methyltetrahydrofuran | 2,2'-methylenebis(5-methyloxolane) |
| 2-Acetylfuran | 2-Acetylfuran |
| Pentane-1,2-diol | Pentane-1,2-diol |
| Pentane-1,4-diol | Pentane-1,4-diol |
| Pentane-1,5-diol | Pentane-1,5-diol |
| 1-Pentanol | Pentan-1-ol |
| 2-Pentanol | Pentan-2-ol |
| Isobutanol | 2-Methylpropan-1-ol |

Similarly, the hydrogenation product that can be obtained from hydrogenating the dehydration product of C6-carbohydrates typically has a structure according to formula (I), where R¹ = H, Me, CH₂OH, CH₂OR⁴, wherein R⁴ is a linear or branched alkyl group having 1-10 carbon atoms or - CH₂Cl; and R² = H, Me, CHO, CH₂OH, -C(O)CH₃, -CH₂O(O)CR³, -CH₂(furan-2-yl), -CH₂(oxolan-2-yl), -CH₂(5-methylfuran-2-yl), -CH₂(5-methyloxolan-2-yl), -CH₂(5-hydroxymethylfuran-2-yl), -CH₂(5-hydroxymethyloxolan-2-yl), - CH₂(5-[R⁵OCH₂]furan-2-yl), -CH₂(5-[R⁵OCH₂]oxolan-2-yl), -CH₂(5-chloromethylfuran-2-yl), or -CH₂(5-chloromethyloxolan-2-yl), and wherein R³ and R₅ individually represent a linear or branched alkyl group having 2-10 carbon atoms.

As used herein, the singular forms "a", "an" and "the" are intended to include the plural forms as well, unless the context clearly indicates otherwise. For instance, the term "a furanic intermediate" as used herein refers to "one or more intermediates" and the term "a hydrogenation product" herein refers to "one or more hydrogenation products". The term "and/or" includes any and all combinations of one or more of the associated listed items. It will be understood that the terms "comprises" and/or "comprising" specify the presence of stated features but do not preclude the presence or addition of one or more other features.

For the purpose of clarity and a concise description features are described herein as part of the same or separate embodiments, however, it will be appreciated that the scope of the invention may include embodiments having combinations of all or some of the features described.

The invention can be illustrated with the following, non-limiting examples.

### Example 1- Organic solvent screening for dehydration step

A solution of 5 wt% xylose in water was prepared and adjusted to pH1 using sulfuric acid. This was combined in a 1:2 ratio with the desired organic solvent. The mixture was heated at 170 °C for 30 minutes with intensive stirring and then cooled to ambient temperature. The organic phase was extracted and analyzed by GCMS to determine the furfural concentration. The aqueous phase was analysed by HPLC to determine residual xylose concentrations. In this way, a range of solvents were screened for their suitability in the dehydration step.

### Example 2 - Organic solvent screening for hydrogenation step

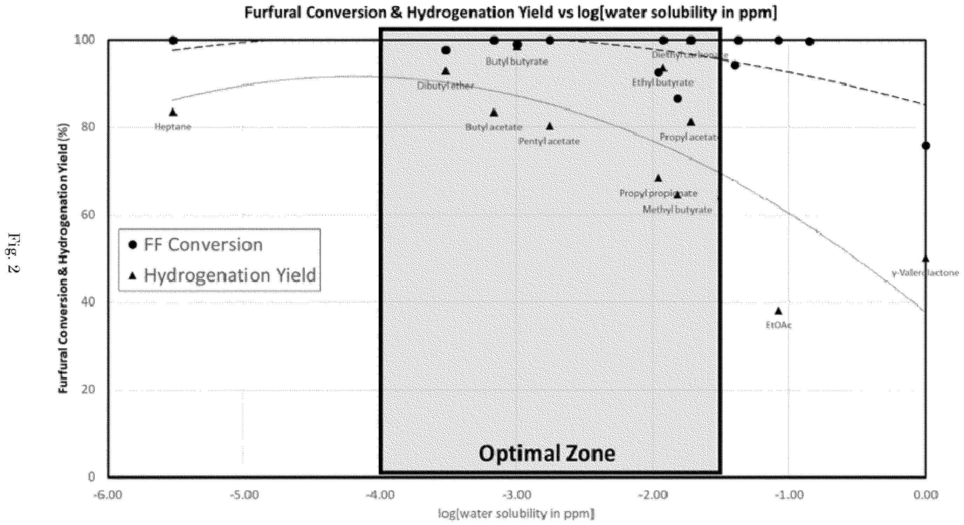

A solution of 1 wt% furfural in the desired organic solvent was prepared and 100 wt% 1% iridium on carbon catalyst was added. The mixture was then reacted at 200 °C for 5 hours with stirring and then cooled to ambient temperature. The mixture was filtered to remove the catalyst and organic phase was extracted and analyzed by GCMS to determine the residual furfural concentration and the concentration of the hydrogenation products. In this way, a range of solvents selected from example 1 were screened for their suitability in the hydrogenation step. The results of this screening are shown in Figure 2.

Hydrogenation yield is determined as the combined yield of furfuryl alcohol and 2-methylfuran. The trendlines in Figure 2 (furfural conversion: --------------; Hydrogenation yield: ················) show that at high log(water solubility in ppm) (i.e. >-1.50), the solvent suitability trends down for both furfural conversion and hydrogenation yield, while at low log(water solubility in ppm) (i.e. <-4.0, the substrate (furfural) is not soluble in the solvent, and an undesired biphasic system is present during hydrogenation.

### Example 3 - Dehydration reaction

A crude, aqueous, hemicelluloses containing solution (containing ~5 wt% xyloses) from a biorefinery was adjusted to pH 1.0 using sulfuric acid. This solution and dibutyl ether were continuously fed into an oscillatory baffled reactor in a 1:2 ratio. The mixture was heated for 30 minutes at 180 °C, while flow oscillations were applied, before leaving the reactor. The process was run until sufficient product sample was prepared. The mixture leaving the reactor was cooled to ambient temperature, filtered and the phases were separated. The crude organic phase was analyzed by GCMS and shown to contain ~2.15 g/L furfural.

### Example 4

The crude organic phase from Example 3 was dried over sodium sulfate, then filtered. The solids were washed with fresh dibutyl ether to yield a dry organic solution. Analysis by GCMS showed this to have a furfural concentration of 0.2 g/L.

### Example 5

The dry organic solution from Example 4 was combined with activated carbon and the mixture was stirred for 2 hours before being filtered. The solids were washed with fresh dibutyl ether to yield a organic solution. Analysis by GCMS showed this to have a furfural concentration of 0.15 g/L.

### Example 6 - Hydrogenation reaction

The dry furfural containing organic solution from Example 4 was fed, together with hydrogen gas, into a reactor that contained a packed bed iridium on carbon particles, with a liquid hourly space velocity (LHSV) of 27. The mixture was heated at 190 °C at a pressure of 80 bar. The resulting solution was cooled to ambient temperature. Time averaged samples over a prolonged period were analyzed by GCMS and showed stable performance, with furfural conversions of up to 86% and a hydrogenation selectivity of up to 78%.

### Example 7 - Hydrogenation reaction

The dry, carbon-washed furfural containing organic solution from Example 5 was fed, together with hydrogen gas, into a reactor that contained a packed bed iridium on carbon particles, with a liquid hourly space velocity (LHSV) of 26. The mixture was heated at 190 °C at a pressure of 80 bar. The resulting solution was cooled to ambient temperature. Time averaged samples over a prolonged period were analyzed by GCMS and showed stable performance, with furfural conversion >99% and a hydrogenation selectivity of up to 62%.

## Claims

1. A process for preparing a furanic intermediate, said process comprising a dehydration step of C5- and/or C6-carbohydrates comprised in a biomass feedstock solution, in the presence of an acid catalyst to form a furanic intermediate, wherein said dehydration step is carried out in a fluid comprising an aqueous solvent and an organic solvent, wherein the organic solvent has a log(water solubility in ppm) at room temperature in the range of -4.0 to -1.5 and a boiling point of less than 210 °C.

2. The process according to claim 1, wherein the concentration of carbohydrates in the biomass feedstock solution is less than 10%, preferably in the range of 1-7%, based on the total weight of biomass feedstock solution.

3. The process according to any of the previous claims, wherein the carbohydrates comprise C5- and/or C6 sugars, preferably C5-carbohydrates, more preferably xylose.

4. The process according to any of the previous claims, wherein the organic solvent has a log(water solubility in ppm) in the range of -3.5 to - 1.65.

5. The process according to any of the previous claims, wherein the organic solvent has a boiling point between 70 and 210 °C, preferably between 100 and 180 °C.

6. The process according to any of the previous claims, wherein the organic solvent is selected from the group consisting of thioethers, ethers, esters, alcohols, carbonates, carboxylic acids, hydrocarbons, and combinations thereof, preferably from the group consisting of ethers, esters, alcohols, and combinations thereof, more preferably from the group consisting of ethers, esters and alcohols having 2 to 10 carbon atoms, even more preferably from the group consisting of symmetrical ethers having 2 to 10 carbon atoms.

7. The process according to any of the previous claims, wherein the organic solvent is selected from the group consisting of ethyl propionate, propyl acetate, diethyl carbonate, ethyl propyl ether, methyl butyrate, methyl furoate, cyclopentyl methyl ether, hexanoic acid, ethylene glycol butyl ether acetate, diisopropyl ether, butyl acetate, ethyl butyrate, propyl propionate, dimethyltetrahydrofuran, tert-butyl acetate, isobutyl acetate, sec-butyl acetate, 1-hexanol, dipropylether, diethyl thioether, dimethyl thioether, tert-amyl methyl ether, methyl benzoate, ethylene glycol dibutyl ether, isoamyl acetate, pentyl acetate, 1-heptanol, 2-octanol, isobutyl isobutyrate, 2-ethylhexanol, 6-methyl-1-heptanol, 1-octanol, hexyl acetate, butylbutyrate, dibutyl ether, 1-pentene, cyclopentane, m-xylene, 1,2-xylene, ethylbenzene, and combinations thereof; preferably from the group consisting of dibutyl ether, butyl acetate, butyl butyrate, pentyl acetate, propyl propionate, ethyl butyrate, methyl butyrate, propyl acetate, diethyl carbonate, and combinations thereof, more preferably wherein the organic solvent is dibutyl ether.

8. The process according to any of the previous claims, wherein the furanic intermediate comprises a compound selected from the group consisting of furfural, 5-methylfurfural, 5-hydroxymethylfurfural (abbreviated as 5-HMF), 5-alkoxymethylfurfural, 5-chloromethylfurfural, and combinations thereof, preferably furfural.

9. A process for preparing a hydrogenation product, said process comprising the dehydration step according to any of the previous claims, which dehydration step is followed by a hydrogenation step of the furanic intermediate to form a hydrogenation product, wherein said hydrogenation step is carried out in a fluid comprising at least part of the organic solvent that was used for the dehydration step.

10. The process according to claim 9, wherein the fluid in which the hydrogenation step is carried out is the same fluid in which the dehydration step was carried out.

11. The process according to claim 9, further comprising a separation step following the dehydration step, wherein the organic solvent comprising at least part of the furanic intermediate is separated from the aqueous solvent, and wherein the separated organic solvent comprising at least part of the furanic intermediate is subsequently used in the hydrogenation step.

12. The process according to any of claims 9-11, wherein the dehydration step is carried out under the following conditions:
- at a temperature between 140 and 220 °C, preferably between 160 and 200 °C;
- for a heating time of between 2 and 60 minutes, preferably between 15 and 45 minutes;
- at a pH of between 2.0 and 0.0, preferably between 0.5 and 1.5; and/or
- wherein the aqueous solvent and the organic solvent are present in a weight ratio of between 5:1 and 1:5, preferably between 3:1 and 1:3; and/or
wherein the hydrogenation of the furanic intermediate is carried out under the following conditions:
- a temperature between 100 and 260 °C, more preferably between 160 and 200 °C;
=- for a heating time of between 0.5 and 300 minutes, more preferably between 1 and 180 minutes;
- in the presence of hydrogen gas at a pressure between 1 and 100 bar, more preferably between 30 and 80 bar; and/or
- in the presence of a catalyst that may be homogenous or heterogeneous, preferably heterogeneous, and/or is preferably selected from the group consisting of gold, platinum, ruthenium, rhodium, palladium, osmium, iridium, silver, copper, mercury, technetium, rhenium, arsenic, antimony, or bismuth, optionally supported on an inert material that is preferably selected from the group consisting of carbon, alumina, silica, titania, zirconia, magnesia, alumino silicates/zeolites, calcium carbonate, sodium phosphate, barium sulfate or phosphate, cerium oxide, europium oxide, gadolinium oxide, montmorillonite, hydrotalcite, kaolinite, polymeric resins, and combinations thereof.

13. The process according to any of claim 9-12, wherein the hydrogenation product is a product according to formula (I), 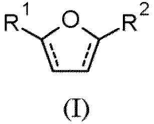 wherein
R¹ = H, Me, CH₂OH, CH₂OR⁴, wherein R⁴ is a linear or branched alkyl group having 2-10 carbon atoms, or CH₂Cl; and
R² = H, Me, CHO, CH₂OH, -C(O)CH₃, -CH₂O(O)CR³, -CH₂(furan-2-yl), - CH₂(oxolan-2-yl), -CH₂(5-methylfuran-2-yl), -CH₂(5-methyloxolan-2-yl), - CH₂(5-hydroxymethylfuran-2-yl), -CH₂(5-hydroxymethyloxolan-2-yl), - CH₂(5-[R⁵OCH₂]furan-2-yl), -CH₂(5-[R⁵OCH₂]oxolan-2-yl), -CH₂(5-chloromethylfuran-2-yl), or -CH₂(5-chloromethyloxolan-2-yl), wherein R³ and R₅ individually represent a linear or branched alkyl group having 2-10 carbon atoms.

14. The process according to any of claim 9-13, wherein the hydrogenation product is a product according to formula (I), 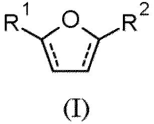 wherein
R¹ = H or Me; and
R² = H, Me, -CHO, -CH₂OH, -CH₂C(O)CH₃, -CH₂CH₂O(O)CR³, wherein R³ is a linear or branched C2- to C10-alkyl, -CH₂(furan-2-yl), -CH₂(oxolan-2-yl), - CH₂(5-methylfuran-2-yl), -CH₂(5-methyloxolan-2-yl), a C5-diol, or a C4- or C5-alcohol, preferably selected from the group consisting of furfural, tetrahydrofurfural, furfuryl alcohol, tetrahydrofurfuryl alcohol, 2-methylfuran, 2-tetrahydrofuran, furan, tetrahydrofuran, furfuryl acetate, tetrahydrofurfuruyl acetate, furfuryl butyrate, tetrahydrofurfuryl butyrate, 2-furfurylfuran, 2-furfuryltetrahydrofuran, 2-tetrahydrofurfuryltetrahydrofuran, 2-furfuryl-5-methylfuran, 2-furfuryl-5-methyltetrahydrofuran, 2-tetrahydrofurfuryl-5-methylfuran, 2-tetrahydrofurfuryl-5-methyltetrahydrofuran, 2-acetylfuran, pentane-1,2-diol, pentane-1,4-diol, pentane-1,5-diol, 1-pentanol, 2-pentanol, and isobutanol, more preferably 2-methylfuran and/or furfuryl alcohol, even more preferably 2-methylfuran.

15. The process according to any of claim 9-13, wherein the hydrogenation product is a product according to formula (I), 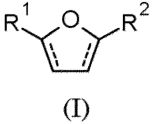
wherein R¹ = H, Me, CH₂OH, CH₂OR⁴, wherein R⁴ is a linear or branched alkyl group having 1-10 carbon atoms or -CH₂Cl; and
R² = H, Me, CHO, CH₂OH, -C(O)CH₃, -CH₂O(O)CR³, -CH₂(furan-2-yl), - CH₂(oxolan-2-yl), -CH₂(5-methylfuran-2-yl), -CH₂(5-methyloxolan-2-yl), - CH₂(5-hydroxymethylfuran-2-yl), -CH₂(5-hydroxymethyloxolan-2-yl), - CH₂(5-[R⁵OCH₂]furan-2-yl), -CH₂(5-[R⁵OCH₂]oxolan-2-yl), -CH₂(5-chloromethylfuran-2-yl), or -CH₂(5-chloromethyloxolan-2-yl), and wherein R³ and R₅ individually represent a linear or branched alkyl group having 2-10 carbon atoms.
